# EUROPÄISCHE PATENTANMELDUNG

(11) **EP 2 123 212 A2**
(43) Veröffentlichungstag der Anmeldung: **25.11.2009**
(21) Anmeldenummer: 09006253.0
(22) Anmeldetag: 08.05.2009
(51) Int. Cl.: A61B 1/045, A61B 1/24

(54) **Kamerahandstück**

(30) Priorität: 24.05.2008 DE 102008024817
(71) Anmelder: Dürr Dental AG, 74321 Bietigheim-Bissingen (DE)
(72) Erfinder: Gebhardt, Herbert, 74936 Siegelsbach (DE); Maier, Raimund, 71732 Tamm (DE)
(74) Vertreter: Ostertag, Reinhard

(57) **Zusammenfassung**

Es wird ein Kamerahandstück angegeben, bei welchem eine korrekte Betätigung eines Auslösemittels (67) durch eine taktil arbeitende Anzeigeeinheit (78) rückgemeldet wird. Diese kann einen umlaufenden Motor (62) und eine durch diesen angetriebene Unwucht (82) umfassen.

## Beschreibung

Die Erfindung betrifft ein Kamerahandstück, insbesondere für dentale und medizinische Zwecke, gemäß dem Oberbegriff des Anspruches 1.

Ein derartiges Kamerahandstück ist in der DE 10 2006 009 177 A1 beschrieben. Bei ihm erfolgt die Quittirung der Betätigung eines ringförmigen Aushilferinges, der vom Kamerahals getragen ist, durch eine akustische Anzeigeeinrichtung.

Akustische Anzeigeeinrichtungen werden oft nicht richtig wahrgenommen, wenn der Umgebungs-Geräuschpegel höher ist oder der das Kamerahandstück bedienende Arzt sich zu sehr darauf konzentriert, einen richtigen Bildausschnitt auszuwählen.

Durch die vorliegende Erfindung soll daher ein Kamerahandstück gemäß dem Oberbegriff des Anspruches 1 so weitergebildet werden, dass die Rückmeldung über die Auslösung eines Bildes sicherer wahrgenommen wird.

Diese Aufgabe ist erfindungsgemäß gelöst durch ein Kamerahandstück mit den im Anspruch 1 angegebenen Merkmalen.

Beim Gebrauch eines erfindungsgemäßen Kamerahandstückes wird der Zahnarzt direkt durch den taktilen Kontakt mit dem Kamerahandstück über das Auslösen eines Bildes informiert. Störende Umwelteinflüsse für diese taktile Benachrichtigung sind nicht gegeben.

Bei Verwendung des erfindungsgemäßen Kamerahandstückes werden die Patienten auch nicht durch Geräusche irritiert, die sie sich nicht erklären können.

Ein weiterer Vorteil des erfindungsgemäßen Kamerahandstückes ist der, dass ein Rechner, an den das Kamerahandstück angeschlossen ist, keine Lautsprecher zu haben braucht.

Vorteilhafte Weiterbildungen der Erfindung sind in Unteransprüchen angegeben.

Bei einem Kamerahandstück gemäß Anspruch 2 erfolgt die Aktivierung des taktilen Alarmes erst nach Ablauf einer vorgegebenen Verzögerungszeit, die in der Regel so gewählt wird, dass das Einfrieren des Kamerabildes beendet ist, bevor die taktile AnzeigeEinrichtung aktiviert wird.

Bei einem Kamerahandstück gemäß Anspruch 3 ist die taktile Rückmeldung zeitlich begrenzt. Die zeitliche Begrenzung kann dann, wenn man das Aktivieren der Anzeigeeinrichtung nicht über ein Verzögerungsglied vornehmen will, so erfolgen, dass die durch die Anzeigeeinrichtung erzeugten Bewegungen so klein sind, dass das Kamerabild nicht beeinträchtigt wird. Über die Länge der Zeit, über welche die Anzeigeeinrichtung aktiviert wird, kann man auch unterschiedlichen taktilen Empfindlichkeiten der das Kamerahandstück benutzenden Personen Rechnung tragen.

Taktile Alarmdauern, wie sie im Anspruch 4 angegeben sind, eignen sich besonders gut dazu, die Aufmerksamkeit des Benutzers zu wecken, ohne ihn in seiner Arbeit zu behindern.

Auch mit der Weiterbildung der Erfindung gemäß Anspruch 5 wird erreicht, dass von der taktilen Anzeigeneinrichtung erzeugte kleine Bewegungen das Bild des Kamerahandstückes nicht beeinträchtigen. Dieses Bild ist nämlich schon eingefroren, bevor die taktile Quittung über die Auslösung des Bildes erfolgt.

Eine taktile Anzeigeeinrichtung, wie sie im Anspruch 6 angegeben ist, lässt sich sehr einfach unter Verwendung bekannter marktüblicher Bauelemente realisieren. Über die Größe der Unwucht kann wieder die Stärke des taktilen Reizes variiert werden, mit welcher die Auslösung eines Bildes gemeldet wird.

Auch eine taktile Anzeigeeinrichtung gemäß Anspruch 7 lässt sich mit bekannten marktgängigen Bauelementen aufbauen. Die Amplitude der taktilen Rückmeldung lässt sich über die Amplitude und Frequenz des Wechselsignales einstellen, mit welchem der Schwinger bzw. ein diesen antreibender Elektromagnet beaufschlagt wird.

Eine taktile Anzeigeeinrichtung, wie sie im Anspruch 8 angegeben ist, zeichnet sich durch besonders einfachen mechanischen Aufbau aus. Sie ist auch über sehr lange Zeiten hinweg wartungsfrei.

Die Weiterbildung der Erfindung gemäß Anspruch 9 ist im Hinblick auf eine gut wahrnehmbare taktile Meldung an die Hand eines Benutzers und im Hinblick auf das Vermeiden von die Bildqualität beeinträchtigenden Erschütterungen des Bildwandlers von Vorteil.

Nachstehend wird die Erfindung anhand eines Ausführungsbeispieles unter Bezugnahme auf die Zeichnung näher erläutert. In dieser zeigen:

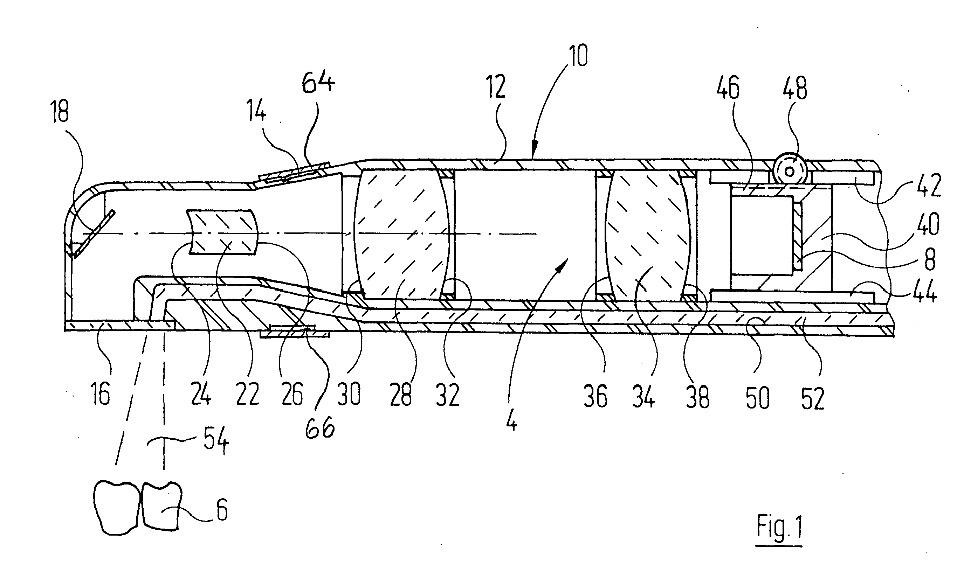

- Figur 1: einen Längsschnitt durch den vorderen Abschnitt eines Kamerahandstückes;
- Figur 2: eine schematische seitliche Ansicht des gesamten Kamerahandstückes zusammen mit einer Schaltung, die zum Übernehmen einzelner Kamerabilder dient; und
- Figur 3: eine ähnliche Darstellung wie Figur 2, in der ein abgewandeltes Kamerahandstück gezeigt ist.

Die in der Zeichnung wiedergegebene dentale Kamera hat ein Gehäuse 10, das als Plastikspritzteil hergestellt ist. Das Gehäuse 10 ist als einstückiges Gehäuse wiedergegeben; es versteht sich, dass der Fachmann es je nach den Herstellungserfordernissen als mehrteiliges Gehäuse konstruieren kann, wobei die verschiedenen Gehäuseteile dann unter Zwischenschaltung von Dichtungen dicht miteinander verbunden werden oder miteinander verklebt oder verschweißt werden.

Das Gehäuse 10 hat einen Griffabschnitt 12, der im wesentlichen die Form einer an den Enden geschlossenen zylindrischen Hülse hat. Der Griffabschnitt 12 trägt an seinem freien Ende einen sich verjüngenden und abgewinkelten Gehäuseabschnitt 14, dessen nach unten gewandtes Ende durch ein Fenster 16 bündig und dicht verschlossen ist.

In dem Gehäuse 10 ist eine insgesamt mit 4 bezeichnete Optik angeordnet, die ein schematisch angedeutetet Objekt 6 (Zahn oder Kieferbogen) auf einen Bildwandler 8 abbildet. Bei dem Bildwandler kann es sich um ein Farb-CCD handeln.

In dem abgewinkelten Teil des Gehäuseabschnittes 14 ist ein Umlenkspiegel 18 angeordnet, der unter 45 Grad zur Achse des Griffabschnittes 12 und zur Achse des Fensters 16 angestellt ist. Auf den Umlenkspiegel 18 folgt in Betrachtungsrichtung (vom Fenster 16 in den Griffabschnitt 12 hinein) eine zylindrische optische Komponente 20 mit planparallelen Stirnflächen (dicke optische Platte mit Stirnflächen 19, 21).

Hinter der Komponente 20 ist eine Linse 22 angeordnet, die eine konkave vordere Stirnfläche 24 und eine konvexe hintere Stirnfläche 26 aufweist. Die Linse 22 bildet zusammen mit der eine dicke optische Platte darstellenden Komponente 20 eine objektseitige Linsenanordnung.

Unter größerem Abstand von der Linse 22 ist eine Zwischenlinse 28 angeordnet, welche eine konvexe objektseitige Stirnfläche 30 und eine konvexe wandlerseitige Stirnfläche 32 aufweist.

Wiederum eine größere Strecke hinter der Zwischenlinse 24 ist eine wandlerseitige Linse 34 vorgesehen, die eine konvexe objektseitige Stirnfläche 36 und eine konvexe wandlerseitige Stirnfläche 38 aufweist.

Der Bildwandler 8 ist auf einem Schlitten 40 angeordnet, der durch auf der Innenseite des Gehäuses 10 vorgesehene Führungsrippen 42, 44 längs der Achse der Optik 4 bewegbar geführt ist. Auf der einen Längsfläche des Schlittens 40 ist eine Zahnstange 46 ausgebildet, die mit einem Zahnrad 48 kämmt, welches drehbar am Gehäuse 10 gelagert ist und mit einem Zahnradabschnitt durch das Gehäuse 10 nach außen übersteht. Durch Drehen des Zahnrades 48 kann somit der Bildwandler 8 längs der Achse der Optik 4 verstellt werden.

In dem Gehäuse 10 ist ein im wesentlichen in axialer Richtung verlaufender Kanal 50 vorgesehen, in welchem ein Lichtleiter 52 vorgesehen ist. Ein Endabschnitt des Kanales 50 und des Lichtleiters 52 sind so abgewinkelt, dass auf den Lichtleiter 52 gegebenes Licht den Lichtleiter 52 leicht geneigt zur Achse des Fensters 16 verlässt, wie bei 54 gezeigt.

Der Bildwandler 8 und der Lichtleiter 52 sind über eine in der Zeichnung nicht wiedergegebene (dort rechts zu denkende) Steckverbindung mit einem Versorgungsschlauch verbunden, der ein zu einer Bildauswerteelektronik führendes Kabel sowie einen weiteren Lichtleiter aufweist, der zu einer Kaltlichtquelle führt.

Auf dem kegelstumpfförmigen Gehäuseabschnitt 40 ist eine ringförmige Membran 64 gezeigt, die aus einem piezoelektrischen Material angefertigt ist und in der Zeichnung nicht näher dargestellte Elektroden trägt. Bei der Unterseite der Membran 64 ist die Außenseite des Gehäuseabschnittes 14 mit einer flachen Ringnut 66 versehen, in welche die Membran 64 bei Kraftbeaufschlagung von außen hineingedrückt werden kann.

Diese Membran 64 stellt so einen ringförmigen Auslöser dar, der benutzt wird, um ein interessierendes Bild des Bildwandlers 8 einzufrieren.

Dies erfolgt so, dass das Ausgangssignal der Membran 64 auf einen Diskriminator 68 gegeben wird, welcher an seinem Ausgang dann ein Signal bereitstellt, wenn das Eingangssignal einen vorgegebenen Schwellwert überschreitet. Durch die Flanke des Ausgangssignales wird dann ein Speicher 70 zum Einlesen getriggert, der mit dem Bildwandler 8 verbunden ist.

Das Ausgangssignal des Diskriminators 68 gelangt über einen Verzögerungkreis 72 und ein Zeitglied 74 auf eine Betriebsschaltung 76, die eine taktile Anzeigeeinheit 78 mit einer geeigneten Speisespannung beaufschlagt.

Beim hier betrachteten Ausführungsbeispiel ist die taktile Anzeigeeinheit 78 als Kombination eines Elektromotors 80 und einer von dessen Welle getragenen Unwucht 82 dargestellt. In diesem Falle kann der Elektromotor 80 z. B. ein Gleichstrommotor sein, der mit einer Frequenz von etwa 175 Hertz umläuft.

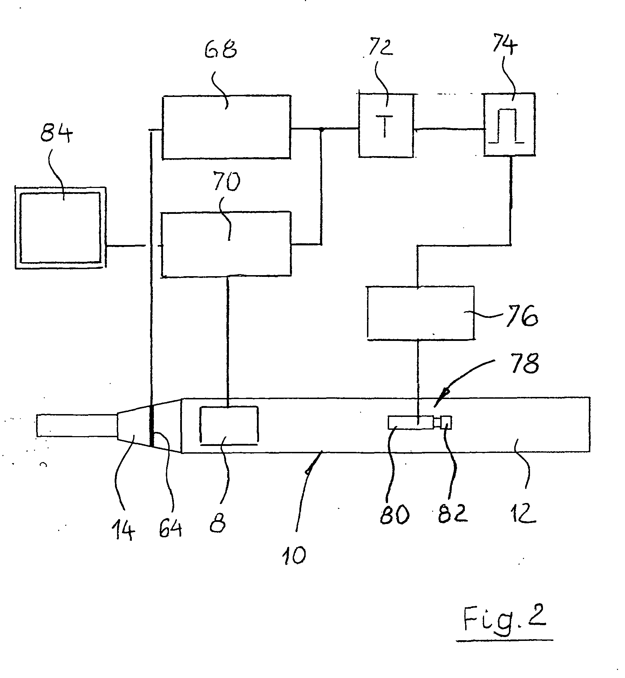

Das in Figur 2 gezeigte Kamerahandstück arbeitet folgendermaßen:

Mit dem Betätigen der Membran 64 wird das gerade vom Bildwandler 8 bereitgestellte Bild in den Speicher 70 übernommen und dort eingefroren. Nach der durch den Verzögerungskreis 72 vorgegebenen Zeit wird das Zeitglied 74 angestoßen, und für die Dauer der Periode des Zeitgliedes 74 und die Betriebsschaltung 76 aktiviert, wodurch die taktile Anzeigeeinheit 78 für eine entsprechende Zeitspanne im Griffteil 18 des Kamerahandstückes von der Hand eines Benutzers taktil wahrnehmbare Signale erzeugt. Auf diese Weise weiß der Benutzer, dass das befohlende Auslösen zu dem gewünschten Speichern eines Bildes geführt hat.

In Abwandlung kann man als taktile Anzeigeeinheit 78 auch einen schwingend gelagerten Anker verwenden, der mit einem Elektromagneten zusammenarbeitet. Dieser wird dann durch die Betriebsschaltung 76 mit einer geeigneten Wechselspannung beaufschlagt, wodurch dann der Anker taktil wahrnehmbar zu schwingen beginnt.

Statt eines eletromagnetischen Schwingers kann man auch einen Festkörperschwinger verwenden, z.B. einen magnetostriktiven oder pizeoelektrischen Schwinger.

Typischerweise ist das Zeitglied 74 auf eine Zeitspanne von 80 bis 200 ms, vorzugsweise 60 bis 100 ms eingestellt. Derartige kurze taktil wahrnehmbare Signale sind als Quittierung für ein vollzogenes Auslösen der Kamera ausreichend.

Der Speicher 70 ist seinerseits an einen Monitor 84 angeschlossen, an dem das durch Auslösen erhaltene Bild betrachtet werden kann.

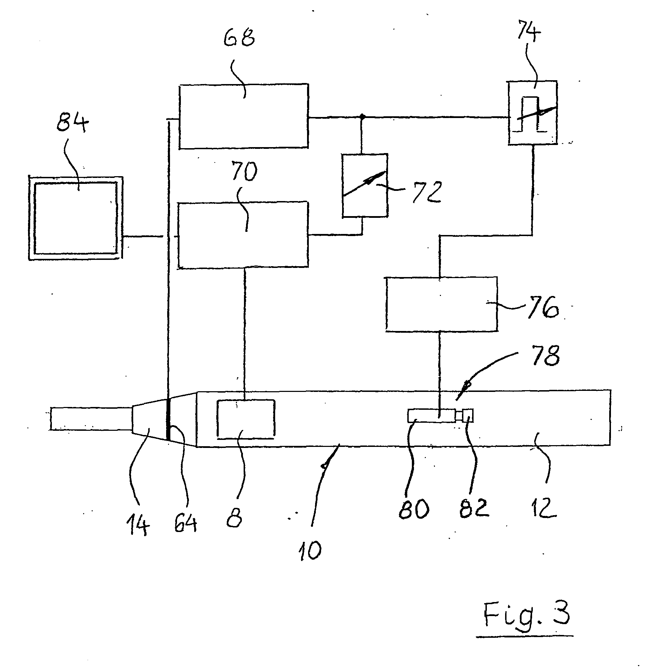

Das Ausführungsbeispiel nach Figur 3 ähnelt dem nach Figur 2. Entsprechende Komponenten tragen wieder die selben Bezugszeichen und brauchen nicht noch einmal detailliert beschrieben zu werden.

Bei diesem Kamerhandstück wird die Anzeigeeinheit 78 unmittelbar mit der Betätigung der Membran 64 eingeschaltet und nach der durch das Zeitglied 74 vorgegebenen kurzen Zeitspanne ("taktiles Klick") wieder ausgeschaltet.

Der Speicher 70 wird über den Verzögerungskreis 72 erst eine vorgegebene Zeitspanne nach Beendigung des Ausgangssignales des Zeitgliedes 74 aktiviert.

Auch auf diese Weise ist sichergestellt, dass die taktile Anzeigeeinheit 78 keine Unschärfe des aufgenommenen Bildes verursacht.

Die Periode des Zeitgliedes 74 und die Verzögerungszeit des Verzögerungskreises 72 können vom Benutzer einstellbar sein, wie in Figur 3 durch Pfeile angedeutet.

## Patentansprüche

1. Kamerahandstück, insbesondere für dentale medizinische Zwecke, mit einer Optik (28), mit einem Bildwandler (8), mit einem Auslösemittel (64), welches die Übernahme eines Bildes des Bildwandlers (8) in einen Speicher (70) steuert, und mit einer durch das Auslösemittel (64) aktivierbaren Anzeigeeinheit (78), **dadurch gekennzeichnet, dass** die Anzeigeeinheit (78) taktil wahrnehmbare Signale erzeugt.

2. Kamerahandstück nach Anspruch 1, **dadurch gekennzeichnet, dass** die Anzeigeeinheit (78) über ein Verzögerungsglied (72) durch das Auslösemittel (64) betätigt wird.

3. Kamerahandstück nach Anspruch 1 oder 2, **dadurch**
**gekennzeichnet, dass** die Anzeigeeinheit (78) über ein Zeitglied (74) erregt wird.

4. Kamerahandstück nach Anspruch 3, **dadurch gekennzeichnet, dass** die Periode des Zeitgliedes (74) etwa 60 ms bis etwa 200 ms, vorzugsweise etwa 80 ms bis etwa 100 ms beträgt.

5. Kamerahandstück nach Anspruch 1 oder 2, **dadurch**
**gekennzeichnet, dass** die Ansteuerung des Speichers (70) durch das Auslösemittel (64) über ein Zeitglied (74) erfolgt.

6. Kamerahandstück nach einem der Ansprüche 1 bis 5, **dadurch gekennzeichnet, dass** die Anzeigeeinheit
(78) eine Unwucht (82) umfasst, die durch einen in Abhängigkeit vom Ausgangssignal des Auslösemittels (64) gesteuerten Antriebsmotor (80) bewegt wird.

7. Kamerahandstück nach einem der Ansprüche 1 bis 5,
**dadurch gekennzeichnet, dass** die taktile Anzeigeeinheit (78) einen Schwinger umfasst, der durch einen in Abhängigkeit vom Ausgangssignal des Auslösemittels (84) gesteuerten Antriebsmotor (84) bewegt wird.

8. Kamerahandstück nach einem der Ansprüche 1 bis 5,
**dadurch gekennzeichnet, dass** die taktile Anzeigeeinrichtung (78) einen Festkörperschwinger, insbesondere einen Piezoschwinger oder einen magnetostriktiven Schwinger umfasst, der in Abhängigkeit vom Ausgangssignal des Auslösemittels (64) aktiviert wird.

9. Kamerahandstück nach einem der Ansprüche 1 bis 8,
**dadurch gekennzeichnet, dass** die Arbeitsfrequenz der taktilen Anzeigeeinheit (80) zwischen 100 und 500, vorzusweise etwa 150 und etwa 250 Hz liegt.
